# FASCICULE DE BREVET EUROPEEN

(11) **EP 1 335 672 B1**
(45) Date de publication et mention de la délivrance du brevet: **05.10.2005**
(21) Numéro de dépôt: 01997261.1
(22) Date de dépôt: 21.11.2001
(51) Int. Cl.: A61B 17/16

(54) **DISPOSITIF DE FORAGE COMPRENANT UN TREPAN RECUPERATEUR D'OS**
BOHRVORRICHTUNG MIT SCHÄDELBOHRER FÜR KNOCHENVERWERTUNG
DRILLING DEVICE COMPRISING A BONE RECUPERATING TREPHINE

(30) Priorité: 23.11.2000 FR 0015131
(43) Date de publication de la demande: 20.08.2003
(73) Titulaire: Kurc, Michel, 75011 Paris (FR)
(72) Inventeur: Kurc, Michel, 75011 Paris (FR)
(74) Mandataire: Orsini, Fabienne
(86) Numéro de dépôt international: PCT/FR2001/003663
(87) Numéro de publication internationale: WO 2002/041792

(56) Documents cités:
- EP-A- 0 992 218
- EP-A- 0 995 402
- US-A- 4 696 308
- US-A- 4 895 146
- US-A- 5 331 972
- US-A- 5 928 238
- US-A- 6 110 178
- US-A- 6 139 509

## Description

La présente invention concerne un dispositif de forage comprenant un trépan récupérateur d'os.

L'invention trouve une application particulièrement avantageuse dans les domaines de la parodontologie, de l'implantologie et de l'orthopédie, où il existe une demande de récupération de l'os d'un patient pour faire des greffes osseuses ou comme matériau de comblement.

De manière classique, le trépan récupérateur d'os se présente sous la forme d'un tube apte à être entraîné en rotation, comprenant, à son extrémité avant, une ouverture munie sur son pourtour de dents. Ce trépan est fermé à son extrémité arrière, est raccordé à un mandrin, pouvant former une seule pièce avec celui-ci, pour son montage sur un contre-angle ou une pièce à main, et est entraîné à basse vitesse de rotation.

L'inconvénient principal d'un tel trépan est que la carotte osseuse prélevée à l'aide de ce dernier ne peut être directement utilisée. Cette carotte doit être retirée du trépan et manipulée avec différents instruments. Ces manipulations de transfert du prélèvement osseux peuvent engendrer malencontreusement une contamination de ce dernier par des agents contaminants externes.

On connaît du document US 5 928 238 un dispositif de forage chirurgical qui comprend un trépan récupérateur d'os classique tel que décrit ci-dessus, traversé longitudinalement par une tige qui porte, à son extrémité libre, une pointe qui s'étend au-delà de ladite ouverture munie de dents du trépan.

Cette pointe s'ancre dans l'os à forer pour éviter que le trépan ne dérape lorsqu'il attaque l'os.

Outre le fait que ce dispositif de forage permet d'obtenir seulement une carotte d'os non broyé, il présente les inconvénients précités liés au trépan récupérateur d'os.

En outre, il existe actuellement différents dispositifs d'obtention d'un broyat osseux. Certains de ces dispositifs comprennent, d'une part, un foret, et, d'autre part, un dispositif de récupération du broyat osseux constitué d'un filtre incorporé à une aspiration chirurgicale.

Lors du forage sous irrigation, l'aspiration chirurgicale est mise en fonctionnement de manière à récupérer les copeaux osseux à la sortie du puits de forage. Ces copeaux osseux qui constituent le broyat sont retenus par le filtre de l'aspiration chirurgicale.

De tels dispositifs de récupération du broyat osseux peuvent, lors de prélèvements osseux à l'intérieur de la cavité buccale, recueillir alors des agents contaminants provenant de la salive.

D'autres dispositifs d'obtention d'un broyat osseux intègrent une lame foreuse et un filtre dans l'aspiration chirurgicale. L'inconvénient principal de tels dispositifs est qu'ils peuvent également récupérer la matière osseuse avec des agents contaminants et sont particulièrement encombrants.

Du fait de leur encombrement, ces dispositifs ne peuvent pas être utilisés pour effectuer des prélèvements osseux à un niveau postérieur de la cavité buccale, mais peuvent opérer seulement à un niveau antérieur.

En outre, de manière générale, de tels dispositifs sont relativement complexes, ce qui augmente leur coût de fabrication.

Enfin, on connaît du document EP 0 992 218 un dispositif de biopsie permettant d'effectuer des prélèvements osseux et comprenant un trépan récupérateur d'os déplaçable à translation, non pas à rotation, et équipé intérieurement d'une lame de broyage d'os qui s'étend longitudinalement selon l'axe du trépan et qui présente une extrémité tranchante s'étendant au-delà de l'ouverture du trépan.

Une fois que le dispositif de biopsie est introduit dans l'os du patient, le trépan est maintenu fixe et la lame de broyage d'os est entraînée en rotation à l'intérieur du trépan pour constituer un broyat osseux.

Du fait que l'extrémité de la lame de broyage s'étend au-delà de l'ouverture du trépan, le trépan et la lame du dispositif de biopsie décrit dans le document EP 0 992 218 ne peuvent intervenir simultanément pour réaliser la carotte de prélèvement osseux.,

En outre, le document EP 0 992 218 (Le préambule de la revendication 1 est basé sur ce document.) décrit un autre dispositif de biopsie comprenant un trépan tubulaire, ouvert aux deux extrémités, équipé intérieure d'une lame solidaire du trépan et dont une extrémité est pourvue d'un bord tranchant qui se situe dans le plan de l'ouverture avant du trépan.

Toutefois, dans ce dispositif de biopsie, la lame utilisée n'est pas une lame de broyage d'os et un tel dispositif permet seulement d'obtenir une carotte d'os non broyé.

Afin de pallier à l'ensemble des inconvénients précités, la présente invention propose un dispositif de forage récupérateur d'os selon la revendication 1.

Ainsi, avantageusement, le trépan récupérateur d'os du dispositif de forage selon l'invention peut être utilisé directement, sans opération de transfert du prélèvement osseux, sur une seringue d'injection d'os pour réimplanter l'os prélevé à un endroit approprié du squelette d'un patient.

A cet effet, selon une caractéristique avantageuse du trépan du dispositif de forage selon l'invention, il est apte à être monté sur l'extrémité du conduit cylindrique, dans lequel coulisse la tige de piston, d'une seringue d'injection d'os par l'intermédiaire de moyens de montage amovible.

En outre, dans le dispositif de forage selon l'invention, le trépan et la lame de broyage d'os interviennent simultanément pour réaliser la carotte de broyat osseux sans que l'os prélevé ne soit contaminé par des agents contaminants et sans provoquer de bouchon à l'entrée du trépan.

A l'aide du dispositif de forage selon l'invention, la carotte prélevée par le trépan est immédiatement broyée par la lame, le broyat osseux remplissant progressivement le tube trépan.

Selon un mode de réalisation préférentiel du dispositif de forage selon l'invention, ladite lame s'étend longitudinalement selon l'axe X du trépan.

Avantageusement, ladite lame peut comporter un pointeau centré entre les deux bords tranchants précités et s'étendant à l'extérieur du trépan de manière à guider le trépan lors d'un forage.

Préférentiellement, cette lame fait partie d'un foret adapté à être monté de manière amovible sur le trépan et comportant un mandrin d'entraînement en rotation, à l'extrémité duquel est monté ie trépan, solidaire de ladite lame, et s'étendant dans un sens opposé à cette dernière.

Ainsi, avantageusement, après avoir prélevé une carotte d'os broyé, le foret peut être désaccouplé du trépan et celui-ci contenant le broyat osseux peut être adapté par des moyens de montage à une seringue d'injection d'os, comme explicité précédemment.

Avantageusement, le mandrin du foret du dispositif de forage précité comporte un alésage d'irrigation interne sur toute la longueur et pouvant se prolonger à l'intérieur d'une ouverture médiane de ladite lame en forme de U.

Selon un mode de réalisation, les moyens de montage amovible du trépan sur le foret sont des moyens à goupille.

Selon un autre mode de réalisation, les moyens de montage amovible du trépan sur le foret sont des moyens à baïonnette.

Selon un autre mode de réalisation, les moyens de montage du trépan sur le foret sont du type à encliquetage.

Selon un autre mode de réalisation, les moyens de montage du trépan sur le foret sont du type à vissage.

Selon un autre mode de réalisation du dispositif de forage selon l'invention, ladite lame s'étend longitudinalement globalement transversalement à l'axe X du trépan.

Ladite lame peut former alors une seule pièce avec le trépan et être située à proximité de l'extrémité avant du trépan.

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue schématique de côté du trépan récupérateur d'os selon l'invention et d'un mandrin d'entraînement à rotation associé ;
- la figure 2 est une vue schématique en perspective du trépan de la figure 1 adapté sur un conduit cylindrique d'une seringue d'injection d'os ;
- la figure 3 est une vue schématique de côté d'un premier mode de réalisation du dispositif de forage selon l'invention ;
- la figure 4 est une vue schématique de côté en éclaté du dispositif de forage de la figure 3 ;
- la figure 5 est une vue schématique de côté d'un deuxième mode de réalisation du dispositif de forage selon l'invention, sur laquelle on visualise par transparence les éléments internes de celui-ci ;
- la figure 6 est une vue schématique de côté en éclaté du dispositif de forage de la figure 5 ;
- la figure 7 est une vue schématique d'un autre côté, vu sur la tranche de la lame, du dispositif de forage de la figure 6 ;
- la figure 8 est une vue schématique d'un troisième mode de réalisation du dispositif de forage selon l'invention, sur laquelle on visualise par transparence les éléments internes de celui-ci ; et
- la figure 9 est une vue schématique de côté en éclaté du dispositif de forage de la figure 8.

En préliminaire, on notera que les éléments identiques ou similaires des différents modes de réalisation de l'invention, qui apparaissent sur les différentes figures, seront, dans la mesure du possible, référencés par les mêmes signes de référence et ne seront pas décrits à chaque fois.

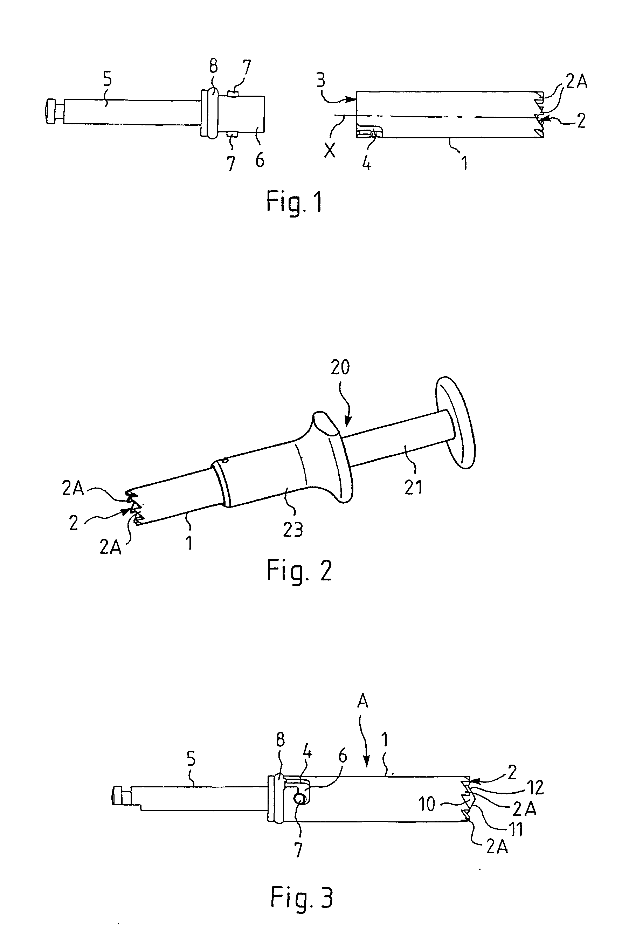

Sur la figure 1, on a représenté un trépan 1 récupérateur d'os qui se présente sous la forme d'un tube d'axe X apte à être entraîné en rotation par l'intermédiaire notamment d'un mandrin 5. Ce trépan 1 comprend, à son extrémité avant, une ouverture 2 munie sur son pourtour de dents 2A permettant lors d'un forage d'attaquer l'os d'un squelette d'un patient pour prélever une carotte osseuse.

En outre, ce trépan 1 comporte une ouverture 3 à son extrémité arrière.

Il comporte, en partie arrière, de manière adjacente à l'ouverture 3, des moyens de montage amovible sur l'extrémité 6 du mandrin 5. Ce mandrin 5 peut être accouplé à un contre-angle ou une pièce à main (non représenté) et être entraîné à basse vitesse de rotation pour effectuer un forage.

Ici, les moyens de montage sont des moyens à baïonnette, comprenant sur le trépan 1 deux gorges 4, positionnées symétriquement par rapport à l'axe X, et débouchant sur le bord de l'ouverture 3 du trépan, ces gorges 4 étant conformées pour permettre le montage à quart de tour de deux plots 7 positionnés en correspondance sur l'extrémité 6 du mandrin 5.

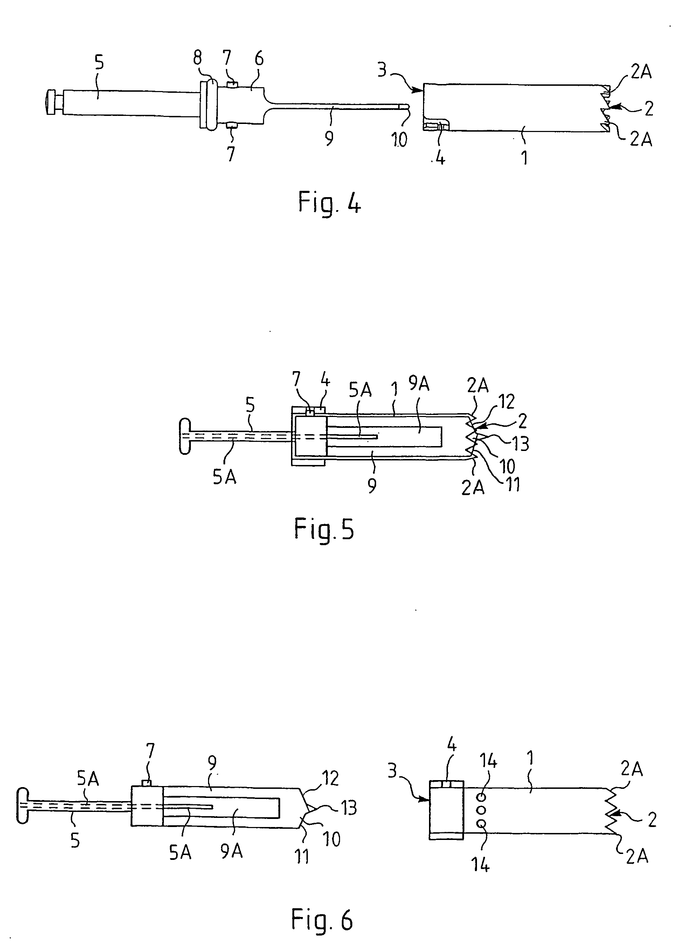

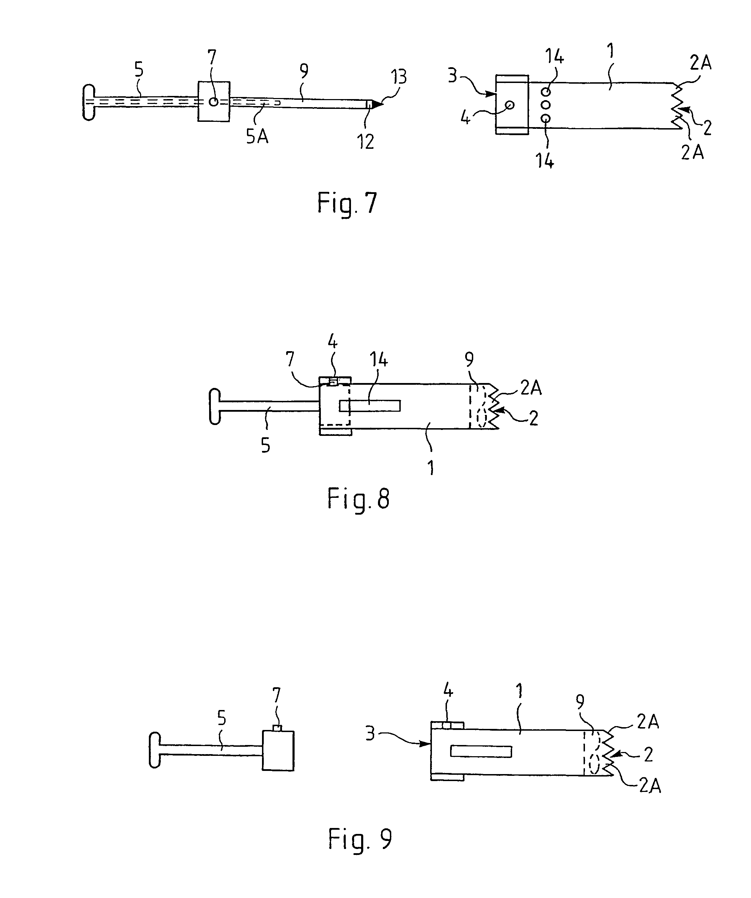

Bien entendu, les moyens de montage amovible du trépan 1 sur le mandrin 5 peuvent, selon d'autres modes de réalisation tels que celui représenté sur les figures 5 et 8, être des moyens à goupille comprenant un logement 4 à l'extrémité arrière du trépan 1 destiné à coopérer avec une goupille 7 prévue à l'extrémité d'un mandrin 5, cette goupille 7 étant actionnée élastiquement à l'aide d'un ressort non représenté.

Selon d'autres variantes non représentées, on peut prévoir que ces moyens de montage sont du type à encliquetage ou à vissage.

En outre, comme cela apparaît sur la figure 1, il est prévu, à la jonction entre la partie arrière du mandrin 5 et son extrémité de montage 6, un joint d'étanchéité 8, ici un joint circulaire, enfilé sur celle-ci de façon à établir, au niveau de l'ouverture arrière 3 du trépan 1, une jonction étanche entre le trépan 1 et le mandrin 5.

Comme cela apparaît plus particulièrement sur la figure 2, avantageusement, le trépan 1 est apte à être monté à l'extrémité d'un conduit cylindrique 23 d'une seringue d'injection d'os 20, par l'intermédiaire de moyens de montage amovible qui sont ici les moyens de montage amovible décrits précédemment pour le montage du trépan 1 sur l'extrémité de montage 6 du mandrin 5.

Le trépan 1 prolonge axialement le conduit cylindrique 23 de la seringue d'injection d'os 20. Cette seringue comporte en outre une tige de piston 21 qui coulisse dans le conduit cylindrique 23 pour injecter dans le site receveur osseux d'un patient le broyat osseux accumulé dans le trépan 1.

De manière générale, les moyens de montage amovible sur l'extrémité du conduit cylindrique d'une seringue d'injection d'os prévus sur le trépan autorisent également son montage sur un mandrin relié à un système d'entraînement en rotation.

Le trépan 1 présente un diamètre externe compris entre environ 3 et 8 millimètres et une longueur égale à environ 20 millimètres pour une utilisation en chirurgie dentaire. Bien entendu, il peut présenter un diamètre et une longueur plus importants pour une utilisation en chirurgie osseuse orthopédique où les prélèvements osseux peuvent être beaucoup plus importants.

Sur les figures 3 et 4, on a représenté plus particulièrement un dispositif de forage A récupérateur d'os qui comprend le trépan 1 représenté sur la figure 1 décrit précédemment et équipé intérieurement d'une lame 9 de broyage d'os.

Cette lame 9 de faible épaisseur s'étend longitudinalement selon l'axe X du trépan et comporte une extrémité 10 pourvue de deux bords tranchants 11, 12 formant une pointe qui s'étend dans le plan de l'ouverture avant 2 du trépan 1.

Cette lame 9 fait partie d'un foret adapté à être monté de manière amovible sur le trépan par des moyens de montage à baïonnette 4, 7, comme décrit précédemment, et comportant un mandrin 5 d'entraînement en rotation, à l'extrémité 6 duquel est monté le trépan 1 de manière amovible, solidaire de ladite lame 9 et s'étendant dans un sens opposé à cette dernière.

Ici, la lame 9 et le mandrin 5 forment une seule pièce.

Tout comme sur la figure 1, il est prévu un joint d'étanchéité 8 enfilé sur l'extrémité 6 du mandrin 5 pour parfaire l'étanchéité au niveau de l'ouverture arrière 3 du trépan 1 lorsque celui-ci est monté sur le mandrin 5.

Sur les figures 5 à 7, on a représenté une variante de réalisation du dispositif de forage représenté sur les figures 3 et 4, selon lequel la lame 9, qui s'étend longitudinalement selon l'axe X du trépan, et qui fait partie d'un foret apte à être monté de manière amovible sur le trépan, se présente sous la forme d'un U dont les branches sont liées à l'extrémité du mandrin 5 et définissent entre elles une ouverture médiane 9A.

Ici, le mandrin 5 est lié temporairement au trépan 1 par des moyens de montage à goupille 4, 7.

En outre, le mandrin 5, représenté sur les figures 5 et 6, comporte un alésage d'irrigation interne 5A sur toute sa longueur qui se prolonge ici à l'intérieur de l'ouverture médiane 9A de ladite lame 9 en forme de U.

A propos d'irrigation, selon une caractéristique avantageuse du trépan 1 selon l'invention, celui-ci peut comporter, à proximité de son extrémité arrière, des alésages d'irrigation 14 tels que des perforations (voir figures 6 et 7) ou des fentes (voir figures 8 et 9).

La lame 9 représentée sur les figures 5 et 6 présente, à son extrémité 10 pourvue des deux bords tranchants 11 et 12, un pointeau 13 centré entre les deux bords tranchants 11 et 12 et s'étendant à l'extérieur du trépan 1 de manière à guider le trépan 1 et plus généralement le dispositif de forage lors d'un forage.

Sur les figures 8 et 9, on a représenté un autre mode de réalisation du dispositif de forage selon l'invention, comportant le trépan 1 contenant une lame 9 qui s'étend longitudinalement globalement transversalement à l'axe X du trépan.

Ici, la lame 9 présente généralement une forme d'hélice, mais elle peut présenter une toute autre forme adaptée telle qu'une forme de croix ou autre.

Le mandrin 5 d'entraînement en rotation est monté de manière amovible sur le trépan 1 à l'aide de moyens de montage à goupille 4, 7 comme décrit précédemment.

La lame 9 forme avantageusement une seule pièce avec le trépan 1 et est située à proximité de l'extrémité avant pourvue de l'ouverture 2 du trépan 1.

Comme cela a déjà été décrit précédemment, à l'arrière, le trépan 1 comporte un alésage d'irrigation 14 se présentant sous la forme de fentes.

Généralement, le trépan 1, son mandrin 5 et la lame 9 sont réalisés en matériau biocompatible tel que par exemple de l'acier trempé inox.

Avantageusement, à l'aide du dispositif de forage représenté sur les différentes figures, la carotte prélevée par le trépan est immédiatement broyée par la lame et le broyat osseux remplit progressivement le trépan.

Le mandrin du dispositif de forage peut être, ultérieurement, après remplissage du trépan, désaccouplé du contre-angle, et le broyat osseux peut être directement injecté en accouplant le trépan à la seringue d'injection d'os 20 représentée sur la figure 2.

Il n'y a plus alors de problème de transfert du broyat osseux et de contamination éventuelle de celui-ci lors d'un tel transfert.

La présente invention n'est nullement limitée aux modes de réalisation décrits et représentés, mais l'homme du métier saura y apporter toute variante conforme aux revendications.

## Revendications

1. Dispositif de forage (A) récupérateur d'os comprenant un trépan (1) se présentant sous la forme d'un tube, ouvert aux deux extrémités, et équipé intérieurement d'une lame (9) solidaire du trépan et dont une extrémité (10) est pourvue de bords tranchants qui s'étend sensiblement dans le plan de l'ouverture avant du trépan (2), **caractérisé en ce que** le trépan est muni de dents (2A) sur le pourtour de son ouverture avant et la lame est apte à broyer l'os pendant la rotation du trépan.

2. Dispositif de forage (A) selon la revendication 1, **caractérisé en ce que** la lame s'étend longitudinalement selon l'axe X du trépan.

3. Dispositif de forage (A) selon l'une des revendications 1 ou 2, **caractérisé en ce que** ladite lame (9) fait partie d'un foret adapté à être monté de manière amovible sur le trépan (1) et comportant un mandrin (5) d'entraînement en rotation, à l'extrémité duquel est monté le trépan (1), solidaire de ladite lame, et s'étendant dans un sens opposé à cette dernière.

4. Dispositif de forage (A) selon la revendication 3, **caractérisé en ce que** ledit mandrin (5) comporte un alésage d'irrigation interne (5A) sur toute sa longueur.

5. Dispositif de forage (A) selon la revendication 4, **caractérisé en ce que** l'alésage d'irrigation (5A) du mandrin (5) se prolonge à l'intérieur d'une ouverture médiane de ladite lame (9) en forme de U.

6. Dispositif de forage (A) selon l'une des revendications 1 à 5, **caractérisé en ce que** ladite lame (9) comporte un pointeau (13) centré entre deux bords tranchants (11, 12) et s'étendant à l'extérieur du trépan (1) de manière à guider le trépan lors d'un forage.

7. Dispositif de forage (A) selon la revendication 1, **caractérisé en ce que** ladite lame (9) s'étend longitudinalement globalement transversalement à l'axe X du trépan.

8. Dispositif de forage (A) selon la revendication 7, **caractérisé en ce que** ladite lame (9) forme une seule pièce avec le trépan (1).

9. Dispositif de forage (A) selon l'une des revendications 3 à 6, **caractérisé en ce que** les moyens de montage amovible du trépan (1) sur le foret sont des moyens à goupille.

10. Dispositif de forage (A) selon l'une des revendications 3 à 6, **caractérisé en ce que** les moyens de montage amovible du trépan sur le foret sont des moyens à baïonnette.

11. Dispositif de forage (A) selon l'une des revendications 3 à 6, **caractérisé en ce que** les moyens de montage du trépan sur le foret sont du type à encliquetage.

12. Dispositif de forage (A) selon l'une des revendications 3 à 6, **caractérisé en ce que** les moyens de montage du trépan sur le foret sont du type à vissage.

13. Dispositif de forage (A) selon l'une des revendications précédentes, **caractérisé en ce que** le trépan (1) comporte, à proximité de son extrémité arrière, des alésages d'irrigation (14) tels que des fentes ou des perforations.

## Patentansprüche

1. Bohrvorrichtung (A) für Knochenverwertung, umfassend einen sich in Form einer Röhre darstellenden Bohrer (1), offen an dem zwei Enden und innseitig mit einer mit dem Bohrer fest verbundenen Klinge (9) ausgerüstet und von der ein Ende (10) mit Schneidrändern versehen ist, sich im wesentlichen in der Ebene der vorderen Öffnung des Bohrers (2) erstreckend, **dadurch** gekennzelchnet, dass der Bohrer mit Zähnen (2A) am Umfang seiner vorderen Öffnung versehen ist und die Klinge ausgelegt ist, den Knochen während der Drehung des Bohrers zu zerkleinern.

2. Bohrvorrichtung (A) nach Anspruch 1, **dadurch gekennzeichnet, dass** die Klinge sich longitudinal entlang der Achse (X) des Bohrers erstreckt.

3. Bohrvorrichtung (A) nach einem der Ansprüche 1 oder 2, **dadurch gekennzeichnet, dass** die Klinge (9) Bestandteil eines Bohrwerkzeugs ist, das dazu ausgelegt ist, in lösbarer Weise an den Bohrer (1) montiert zu werden und eine Spindel (S) für Drehantrieb umfasst, an deren Ende der Bohrer (1) montiert ist, mit der Klinge fest verbunden ist und sich in einer dazu entgegen gesetzten Richtung erstreckt.

4. , Bohrvorrichtung (A) nach Anspruch 3, **dadurch gekennzeichnet, dass** die Spindel (5) eine Innenbewässerungsbohrung (5A) über ihre gesamte Länge aufweist.

5. Bohrvorrichtung (A) nach Anspruch 4, **dadurch gekennzeichnet, dass** die Bewässerungsbohrung (5A) der Spindel (5) sich im Innem einer Mittelöffnung der Klinge (9) in U-Form verlängert.

6. Bohrvorrichtung (A) nach einem der Ansprüche 1 bis 5, **dadurch gekennzeichnet, dass** die Klinge (9) eine zwischen zwei Schneidrändern (11, 12) zentrierten Körner (13) umfasst, der sich außerhalb des Bohrers (1) erstreckt, um den Bohrer während einer Bohrung zu führen.

7. Bohrvorrichtung (A) nach Anspruch 1, **dadurch gekennzeichnet, dass** die Klinge (9) sich in longitudinaler Richtung überwiegend quer zur Achse X des Bohrers erstreckt.

8. Bohrvorrichtung (A) nach Anspruch 7, **dadurch gekennzeichnet, dass** die Klinge (9) mit dem Bohrer (1) ein Stück bildet.

9. Bohrvorrichtung (A) nach einem der Ansprüche 3 bis 6, **dadurch gekennzeichnet, dass** die lösbaren Montagemittel des Bohrers (1) an dem Bohrwerkzeug Stiftmittel sind.

10. Bohrvorrichtung (A) nach einem der Ansprüche 3 bis 6, **dadurch gekennzeichnet, dass** die lösbaren Montagemittel des Bohrers an dem Bohrwerkzeug Bajonettmittel sind.

11. Bohrvorrichtung (A) nach einem der Ansprüche 3 bis 6, **dadurch gekennzeichnet, dass** die Montagemittel des Bohrers an dem Bohrwerkzeug vom Einrasttyp sind.

12. Bohrvorrichtung (A) nach einem der Ansprüche 3 bis 6, **dadurch gekennzeichnet, dass** die Montagemittel des Bohrers an dem Bohrwerkzeug vom Schraubtyp sind.

13. Bohrvorrichtung (A) nach einem der vorangehenden Ansprüche, **dadurch gekennzeichnet, dass** der Bohrer (1) in der Nähe seiner hinteren Öffnung Bewässerungsbohrungen (14), wie Schlitze oder Perforationen, umfasst.

## Claims

1. A bone-recovery drilling device (A) comprising a trephine (1) in the form of a tube, open at both ends, and fitted internally with a blade (9) secured to the trephine and having one end (10) provided with cutting edges that extend substantially in the plane of the front opening of the trephine (2), the device being **characterized in that** the trephine is provided with teeth (2A) on the periphery of its front opening, and the blade is suitable for grinding bone during rotation of the trephine.

2. A drilling device (A) according to claim 1, **characterized in that** the blade extends longitudinally along the axis X of the trephine.

3. A drilling device (A) according to claim 1 or claim 2, **characterized in that** said blade (9) is part of a cutter adapted to be removably mounted on the trephine (1) and further comprising a rotary drive stem (5) having the trephine (1) mounted at the end thereof, the stem being secured to said blade and extending in an opposite direction thereto.

4. A drilling device (A) according to claim 3, **characterized in that** said stem (5) includes an internal irrigation bore (5A) over its entire length.

5. A drilling device (A) according to claim 4, **characterized in that** the irrigation bore (5A) of the stem (5) extends inside a middle opening of said U-shaped blade (9).

6. A drilling device (A) according to any one of claims 1 to 5, **characterized in that** said blade (9) includes a spike (13) centered between two touching edges (11, 12) and projecting outside the trephine (1) so as to guide the trephine during drilling.

7. A drilling device (A) according to claim 1, **characterized in that** said blade (9) extends longitudinally generally transversely to the axis X of the trephine.

8. A drilling device (A) according to claim 7, **characterized in that** said blade (9) is in one piece with the trephine (1).

9. A drilling device (A) according to any one of claims 3 to 6, **characterized in that** the means for removably mounting the trephine (1) on the cutter are pin means.

10. A drilling device (A) according to any one of claims 3 to 6, **characterized in that** the means for removably mounting the trephine on the cutter are bayonet means.

11. A drilling device (A) according to any one of claims 3 to 6, **characterized in that** the means for mounting the trephine on the cutter are of the snap-fastening type.

12. A drilling device (A) according to any one of claims 3 to 6, **characterized in that** the means for mounting the trephine on the cutter are of the screw type.

13. A drilling device (A) according to any preceding claim, **characterized in that**, in the vicinity of its rear end, the trephine (1) includes irrigation bores (14) such as slots or perforations.
